# EUROPÄISCHE PATENTANMELDUNG

(11) **EP 4 449 983 A1**
(43) Veröffentlichungstag der Anmeldung: **23.10.2024**
(21) Anmeldenummer: 23168273.3
(22) Anmeldetag: 17.04.2023
(51) Int. Cl.: A61B 5/055, A61B 5/08, A61B 5/1455, A61B 5/00, A61B 6/00, G01R 33/54, G01R 33/567

(54) **VERFAHREN ZUR VORHERSAGE DER ATEMANHALTEDAUER IN EINEM MEDIZINISCHEN BILDGEBUNGSVERFAHREN**

(71) Anmelder: Siemens Healthineers AG, 91301 Forchheim (DE)
(72) Erfinder: Zeller, Mario, 91054 Erlangen (DE)
(74) Vertreter: Siemens Healthineers Patent Attorneys

(57) **Zusammenfassung**

Ein Verfahren zum Konfigurieren eines Messprotokolls eines medizinischen Bildgebungsverfahrens umfasst die folgenden Schritte. Im Vorfeld einer Datenerfassungsphase des medizinischen Bildgebungsverfahrens werden physiologische Daten einer Untersuchungsperson bereitgestellt. Anschließend wird unter Verwendung der physiologischen Daten eine Atemanhaltedauer während einer Datenerfassungsphase des medizinischen Bildgebungsverfahrens für die Untersuchungsperson vorhergesagt. Diese Atemanhaltedauer entspricht einer Zeitdauer, während der die Untersuchungsperson ihre Atmung auf Anweisung anhalten soll. Schließlich wird unter Verwendung der vorhergesagten Atemanhaltedauer zumindest ein Parameter des Messprotokolls konfiguriert.

## Beschreibung

Die vorliegende Erfindung betrifft das Gebiet der medizinischen Bildgebung, insbesondere die Konfiguration und Durchführung eines Messprotokolls für bildgebende Verfahren, wie beispielsweise Magnetresonanztomographie (MRT), unter Berücksichtigung der Atemanhalte-Fähigkeit einer Untersuchungsperson.

### Technischer Hintergrund

In vielen medizinischen Bildgebungsverfahren, wie beispielsweise bei der Magnetresonanztomographie, ist es notwendig, dass die Untersuchungsperson während bestimmter Phasen des Verfahrens die Atmung anhält, um die Bildqualität zu verbessern und Bildartefakte zu vermeiden. Dies ist insbesondere bei abdominalen Untersuchungen der Fall. In der Praxis werden solche Aufnahmen häufig in mehrere Teilabschnitte unterteilt, die jeweils von Atemanweisungen unterbrochen werden.

Ein technisches Problem besteht darin, die Atemanhaltefähigkeit der Untersuchungsperson bereits vor dem Start der Bildgebung richtig vorherzusagen. Eine überschätzte Atemanhalte-Dauer führt zu verstärkten Bildartefakten, wenn die Untersuchungsperson abrupt einatmen muss, während eine unterschätzte Dauer in unnötigen zusätzlichen Teilabschnitten und damit längerer Messdauer resultiert.

Nach einem herkömmlichen Ansatz wird die Atemanhaltekapazität durch Schwellwert-Verfahren oder maschinelles Lernen ermittelt, indem ein Probescan durchgeführt wird, bei dem die Untersuchungsperson gebeten wird, die Atmung für eine gewisse Zeit anzuhalten, während eine Atemkurve aufgezeichnet wird. Anschließend wird mittels eines Schwellwert-Verfahrens oder Machine Learning-Algorithmus die Dauer ermittelt, in der die Atemkurve flach war, d.h. der Atem angehalten wurde. Dieses Verfahren weist jedoch einige Nachteile auf, wie die Verlängerung der Aufnahmezeit durch den Probescan, zusätzliche Belastung für die Untersuchungsperson und die Begrenzung auf die Erfassung einer der beiden Atemanhalte-Dauern (Inspiration oder Exspiration).

### Zusammenfassung

Daher besteht ein Bedarf an einer verbesserten Lösung zur Vorhersage der Atemanhaltefähigkeit der Untersuchungsperson, die die genannten Nachteile des Standes der Technik überwindet und eine genauere Vorhersage der Atemanhalte-Dauern ermöglicht, ohne die Notwendigkeit eines zusätzlichen Probescans.

Diese Aufgabe wird mit den Merkmalen der unabhängigen Ansprüche gelöst. In den abhängigen Ansprüchen sind weitere vorteilhafte Ausführungsbeispiele beschrieben.

Im Folgenden wird die erfindungsgemäße Lösung in Bezug auf die beanspruchten Verfahren und die beanspruchten medizinischen Bildgebungssysteme, als auch in Bezug auf erfindungsgemäße Rechenvorrichtungen, Computerprogramme, und computerlesbare Datenträger beschrieben. Merkmale, Vorteile oder alternative Ausführungsbeispiele können den jeweils anderen Kategorien zugeordnet werden, und umgekehrt. In anderen Worten, die Ansprüche für medizinische Bildgebungssysteme können durch Merkmale verbessert werden, die im Rahmen der Verfahren beschrieben sind und/oder beansprucht werden, und umgekehrt kann das Verfahren beliebige Schritte oder Merkmale umfassen, die im Rahmen der medizinischen Bildgebungssysteme beschrieben sind.

Ein Verfahren zum Konfigurieren eines Messprotokolls eines medizinischen Bildgebungsverfahrens beinhaltet die folgenden Schritte.

In einem Schritt werden im Vorfeld einer Datenerfassungsphase, in anderen Worten zeitlich (optional ausschließlich) vor der Datenerfassungsphase, in welcher die Bild(roh-)Daten des medizinischen Bildgebungsverfahrens erfasst werden, physiologischen Daten einer Untersuchungsperson erfasst und/oder zur Verarbeitung bereitgestellt.

Eine Datenerfassungsphase eines medizinischen Bildgebungsverfahrens bezieht sich auf einen Zeitraum, in dem das Bildgebungssystem tatsächlich Daten, z.B. Rohdaten, sammelt, um Bilder einer Untersuchungsperson zu erstellen. Während dieser Phase werden die für die Bildgebung erforderlichen Signale, wie z.B. Magnetresonanzsignale in einem MRT-Verfahren oder Röntgenstrahlen in einem CT-Verfahren, erzeugt und von den Detektoren des Bildgebungssystems erfasst.

Die Datenerfassungsphase ist ein kritischer Teil des gesamten medizinischen Bildgebungsprozesses, da sie die Qualität und Genauigkeit der resultierenden Bilder direkt beeinflusst. Um optimale Ergebnisse zu erzielen, müssen verschiedene Faktoren während der Datenerfassungsphase berücksichtigt und kontrolliert werden, wie z.B. die Wahl der richtigen Parametern eines Messprotokolls des medizinischen Bildgebungsverfahrens sowie die Minimierung von Bewegungsartefakten durch geeignete Atemanhalte- oder Bewegungskompensationsstrategien.

Atemanhalte- oder Bewegungskompensationsstrategien sind Techniken, die in medizinischen Bildgebungsverfahren eingesetzt werden, um Bildartefakte aufgrund von Untersuchungspersonenbewegungen, insbesondere Atembewegungen, zu minimieren. Diese Strategien sind entscheidend, um eine hohe Bildqualität und Genauigkeit der resultierenden Bilder zu gewährleisten.

Eine gängige Methode zur Bewegungskompensation ist die Verwendung von Anweisungen zum Anhalten der Atmung, bei denen die Untersuchungsperson aufgefordert wird, ihre Atmung für eine bestimmte Zeitdauer, die sogenannte Atemanhaltedauer, anzuhalten (d.h. zu unterbrechen). Durch das zeitlich begrenzte Anhalten der Atmung während der Datenerfassung können Atembewegungen und damit verbundene Bildartefakte reduziert werden.

Ein Atemanhalteanweisung ist eine Anweisung, die der Untersuchungsperson während des Bildgebungsverfahrens gegeben wird, um das Anhalten der Atmung zu koordinieren. Diese Anweisungen können mündlich oder akustisch übermittelt werden und enthalten in der Regel Anweisungen zur Inspiration (Einatmen) oder Exspiration (Ausatmen) vor dem eigentlichen Anhalten der Atmung.

Die Atemanhaltedauer ist die Zeitdauer, während der die Untersuchungsperson ihre Atmung auf Anweisung anhalten soll. Die optimale Atemanhaltedauer variiert je nach individueller Atemkapazität der Untersuchungsperson und der Bildgebungstechnik. Um die beste Bildqualität und den höchsten Patientenkomfort zu gewährleisten, ist es wichtig, die Atemanhaltedauer korrekt vorherzusagen und das Messprotokoll entsprechend anzupassen.

In einem Schritt wird unter Verwenden der physiologischen Signale, in anderen Worten basierend auf den physiologischen Signalen, eine Atemanhaltedauer für die Untersuchungsperson vorhergesagt. Die Atemanhaltedauer entspricht einer Zeitdauer, während der die Untersuchungsperson während der Datenerfassungsphase des medizinischen Bildgebungsverfahrens ihre Atmung auf Anweisung anhalten soll. die Untersuchungsperson erhält Anweisungen, ihren Atem anzuhalten, und während der Atemanhaltedauer, d.h. ab einer Start-Anweisung, für die Länge der Atemanhaltedauer, bis zu einer Ende-Anweisung, keine Atmung durchzuführen. Die Atemanhaltedauer wird also im Vorfeld der Datenerfassungsphase geschätzt, wobei die bereitgestellten physiologischen Daten der Untersuchungsperson als Grundlage verwendet werden, um die die Atemanhaltedauer festzulegen oder abzuschätzen, die die Untersuchungsperson voraussichtlich auf Anweisung ihren Atem anhalten kann, bevor er wieder Atembewegungen macht.

In einem Schritt wird zumindest ein Parameters des Messprotokolls unter Verwendung der vorhergesagten Atemanhaltedauer, in anderen Worten basierend auf der vorhergesagten Atemanhaltedauer, konfiguriert, d.h. festgelegt.

Die beanspruchte Lösung bietet eine Reihe von technischen Vorteilen gegenüber herkömmlichen Lösungen zur Konfiguration von Messprotokollen und Durchführung von medizinischen Bildgebungsverfahren basierend auf den Messprotokollen. Durch die Verwendung von physiologischen Daten der Untersuchungsperson, zur Vorhersage der Atemanhaltedauer können sowohl Atemanhaltedauern nach Inspiration als auch nach Exspiration genauer vorhergesagt werden, als es durch kontrolliertes und beobachtetes Atemanhalten in einem Probedurchlauf durch die Untersuchungsperson möglich ist. Dies führt zu einer präziseren Anpassung der Parameter des Messprotokolls, was wiederum die Bildqualität verbessert und somit technische Einschränkungen der konventionellen Hardwaresysteme überwindet.

Ein wesentlicher Vorteil der beanspruchten Lösung besteht darin, dass die Vorhersage der Atemanhaltedauer ohne einen dedizierten Probescan erfolgt, wodurch die Messzeit nicht verlängert wird. Dies ermöglicht eine effizientere Untersuchung und trägt dazu bei, die Auslastung der medizinischen Bildgebungssysteme zu optimieren. Eine kürzere Messzeit kann auch zu Kosteneinsparungen führen, da mehr Patienten in kürzerer Zeit untersucht werden können. Auch durch eine exaktere Vorhersage von Atemanhaltedauern kann vermieden werden, dass fehlerhafte Bildgebungen wiederholt werden müssen. Zudem kann die Bildqualität und die Zeitdauer der Untersuchung, welche durch die Atemhalteanweisungen beeinflusst werden, gegeneinander abgewogen werden, um ein optimales Untersuchungsergebnis bereitzustellen.

Ein weiterer Vorteil der beanspruchten Lösung besteht darin, dass die Verwendung von physiologischen Daten die Notwendigkeit zusätzlicher Schritte, den Aufwand und Zeit von Bedienpersonal des Systems, und somit den Verbrauch von Hardware-Ressourcen minimiert. Da die beanspruchte Lösung auf physiologischen Sensoren und der verfügbaren Rechenleistung basiert, kann sie leicht in bestehende medizinische Bildgebungssysteme integriert werden. Dies führt zu einer kosteneffizienteren Lösung im Vergleich zu herkömmlichen Verfahren.

Somit bietet die beanspruchte Erfindung eine verbesserte Effizienz, Geschwindigkeit und Kosteneffektivität im Vergleich zu herkömmlichen Lösungen zur Konfiguration von Messprotokollen und Durchführung von medizinischen Bildgebungsverfahren. Durch die Nutzung von physiologischen Daten kann eine präzisere Anpassung der Messprotokollparameter erreicht werden.

Im Kontext dieses Verfahrens sind physiologische Daten Informationen über den Körper und die Körperfunktionen der Untersuchungsperson. Ein Messprotokoll ist ein Plan oder eine Vorgabe für die Durchführung eines medizinischen Bildgebungsverfahrens.

Das Bereitstellen der physiologischen Daten kann das kontinuierliche Erfassen von physiologischen Signalen der Untersuchungsperson umfassen.

Physiologische Signale sind kontinuierliche erfasste Messwerte, die den Zustand oder die Funktion des Körpers der Untersuchungsperson beschreiben.

Kontinuierlich erfasste physiologische Signale sind Messwerte, die den aktuellen physiologischen Zustand einer Untersuchungsperson widerspiegeln und kontinuierlich überwacht werden. Diese Signale können verschiedene Aspekte der Körperfunktionen wie Atmung, Herzfrequenz, Sauerstoffsättigung oder andere Parameter abbilden. Sie werden in der Regel von speziellen Sensoren erfasst, die entweder direkt an einer Untersuchungsperson angebracht sind oder in dessen Nähe positioniert werden. Physiologische Signale können somit als Zeitreihen von Messwerten definiert werden, die Informationen über den physiologischen Zustand einer Untersuchungsperson liefern. Diese Signale können kontinuierlich oder in diskreten Zeitabständen erfasst werden.

Die erfassten analogen physiologischen Signale werden in der Regel durch Analog-Digital-Wandler (ADC) in digitale Werte umgewandelt. Diese digitalen Werte werden dann gesampelt, das heißt, sie werden in regelmäßigen Abständen aufgezeichnet, um eine zeitliche Folge von Messwerten zu erstellen. Die gesampelten Daten können in Echtzeit analysiert und überwacht werden, um Informationen über den aktuellen Zustand der Untersuchungsperson zu erhalten. In dem offenbarten Verfahren können physiologische Signale in jeder Form als Basis für die Vorhersage der Atemanhaltefähigkeit verwendet werden, beispielsweise können physiologische Signale verschiedener Sensoren in gleichen Zeitabschnitten, oder in teilweise überlappenden Zeitabschnitten, oder auch unterschiedlichen Zeitabschnitten verwendet werden.

Die kontinuierlich erfassten physiologischen Signale können direkt vom Sensor oder aus einem Zwischenspeicher abgerufen werden. In einigen Fällen werden die Signale drahtlos an ein Überwachungsgerät oder eine zentrale Steuerungseinheit übertragen, während in anderen Fällen eine kabelgebundene Verbindung verwendet wird.

Das kontinuierliche Erfassen von physiologischen Signalen ermöglicht eine genauere Vorhersage der Atemanhaltedauer und eine bessere Anpassung des Messprotokolls, was zu einer verbesserten Bildqualität führt.

Die physiologischen Signale können in einer Präparationsphase des medizinischen Bildgebungsverfahrens erfasst werden.

Die Präparationsphase kann sich auf einen Zeitraum vor der eigentlichen Datenerfassung, in dem die Untersuchungsperson und das medizinische Bildgebungssystem für die Untersuchung vorbereitet werden.

Durch das Erfassen der physiologischen Signale in der Präparationsphase kann die Atemanhaltedauer ohne zusätzlichen Zeitbedarf vorhergesagt werden, was zu einer besseren Bildqualität und einer reduzierten Belastung für die Untersuchungsperson führt.

Weitere physiologische Signale können während der Datenerfassungsphase des medizinischen Bildgebungsverfahrens erfasst werden, und der zumindest eine Parameter des Messprotokolls kann basierend auf den weiteren physiologischen Signalen angepasst werden. Ein Anpassen kann ein iteratives Wiederholen der Schritte des Verfahrens zum Konfigurieren des Messprotokolls umfassen, so dass auf Änderungen in der Physiologie der Untersuchungsperson während des medizinischen Bildgebungsverfahrens Rücksicht genommen werden kann.

Das Erfassen weiterer physiologischer Signale während der Datenerfassungsphase und die Anpassung des Messprotokolls basierend auf diesen Signalen ermöglichen zudem eine noch genauere Anpassung des Messprotokolls und führen zu einer verbesserten Bildqualität.

Der Untersuchungsperson kann für das kontinuierliche Erfassen der physiologischen Signale keine Anweisung zum Anhalten der Atmung bereitgestellt werden. In anderen Worten, während der Erfassung der physiologischen Daten werden der Untersuchungsperson keine speziellen Atemkommandos gegeben werden, um die Atmung anzuhalten.

Indem die Untersuchungsperson während des kontinuierlichen Erfassens der physiologischen Signale nicht aufgefordert wird, ihre Atmung anzuhalten, wird der natürliche Atemrhythmus der Untersuchungsperson beibehalten, was zu einer genaueren Vorhersage der Atemanhaltedauer und einer besseren Anpassung des Messprotokolls führt. Jedoch ist auch denkbar, das der Untersuchungsperson Anweisungen gegeben werden, wie er atmen soll, auch kürzere Atemanhalteanweisungen sind denkbar. Die physiologischen Signale können unter Verwendung eines oder mehrerer Sensoren, die an oder in der Nähe der Untersuchungsperson angeordnet sind, erfasst werden. Sensoren sind Geräte oder Instrumente, die physiologische Signale messen und aufzeichnen können. Die Anordnung der Sensoren an oder in der Nähe der Untersuchungsperson ermöglicht eine genaue Erfassung der physiologischen Signale.

Das medizinische Bildgebungsverfahren kann ein Magneteresonanztomographie (MRT)-Verfahren sein. Die physiologischen Signale können unter Verwendung eines Pilot-Tone-Messverfahrens in einem Frequenzbereich erfasst werden, der außerhalb des für die Erfassung von Bilddaten verwendeten Frequenzbereichs liegt.

Das Pilot-Tone-Messverfahren ist eine Technik zur Erfassung von physiologischen Signalen in einem Frequenzbereich, der von dem für die Erfassung von Bilddaten verwendeten Frequenzbereich getrennt ist.

Durch die Verwendung des Pilot-Tone-Messverfahrens zur Erfassung der physiologischen Signale wird eine störungsfreie und genaue Erfassung ermöglicht, was zu einer verbesserten Vorhersage der Atemanhaltedauer und einer besseren Anpassung des Messprotokolls führt.

Die physiologischen Daten können eins oder mehrere umfassen von: Daten aus einer Patientenakte, Messwerte oder Rohdaten von vorhergehenden Untersuchungen an der Untersuchungsperson, und Daten von einer Smart Watch.

Patientenakten enthalten Informationen über die Untersuchungsperson und seine medizinischen Vorgeschichte. Messwerte oder Rohdaten von vorhergehenden Untersuchungen sind Informationen, die bei früheren medizinischen Untersuchungen der Untersuchungsperson erfasst wurden. Daten von einer Smart Watch sind Informationen, die von einem tragbaren elektronischen Gerät erfasst wurden, das die Untersuchungsperson trägt.

Durch die Verwendung dieser zusätzlichen Datenquellen können die Vorhersagegenauigkeit der Atemanhaltedauer und die Anpassung des Messprotokolls weiter verbessert werden, was zu einer besseren Bildqualität und einer reduzierten Belastung für die Untersuchungsperson führt.

Das Vorhersagen der Atemanhaltedauer für die Untersuchungsperson kann das Anwenden eines neuronalen Netzwerks umfassen, das auf der Grundlage von physiologischen Trainingsdaten der Untersuchungsperson trainiert wurde. Eine Eingabe in das neuronale Netzwerk umfasst zumindest die physiologischen Daten, und eine Ausgabe des neuronalen Netzwerks umfasst die Atemanhaltedauer.

Physiologische Trainingsdaten können Daten umfassen, die während des Trainings des neuronalen Netzwerks verwendet werden, um das Netzwerk auf die Vorhersage der Atemanhaltedauer zu trainieren, beispielsweise bekannte Atemanhaltedauern und bekannte physiologische Daten.

Eine Verwendung eines neuronalen Netzes zur Vorhersage der Atemanhaltedauer ermöglicht eine genauere und automatisierte Vorhersage der optimalen Länge der Atemanhaltedauer, was wiederum die Effizienz des Verfahrens erhöht. Darüber hinaus kann das neuronale Netz beispielsweise kontinuierlich mit neuen Daten trainiert werden, was dazu führt, dass die Vorhersagegenauigkeit im Laufe der Zeit verbessert wird. Dies bietet einen zusätzlichen Vorteil gegenüber herkömmlichen Methoden, bei denen die Vorhersagegenauigkeit möglicherweise nicht mit der Zeit verbessert werden kann.

Die physiologischen Daten können in Bilddaten konvertiert werden und als Bilddaten in das neuronale Netzwerk eingegeben werden.

Bilddaten sind visuelle Darstellungen von Informationen, die zur Verarbeitung durch das neuronale Netzwerk verwendet werden können. Die Konvertierung von physiologischen Daten in Bilddaten ermöglicht eine effiziente Verarbeitung der Informationen durch das neuronale Netzwerk.

Durch die Verwendung von Bilddaten zur Eingabe in das neuronale Netzwerk kann die Vorhersagegenauigkeit der Atemanhaltedauer weiter verbessert werden, was zu einer besseren Bildqualität und einer reduzierten Belastung für die Untersuchungsperson führt.

Das neuronale Netzwerk kann zumindest ein Convolutional Neural Network (CNN) oder ein Recurrent Neural Network (RNN) umfassen. Ein CNN ist eine Art von neuronalem Netzwerk, das speziell für die Verarbeitung von Bilddaten entwickelt wurde. Ein RNN ist eine Art von neuronalem Netzwerk, das für die Verarbeitung von sequentiellen Daten, wie Zeitreihendaten, entwickelt wurde. Die Verwendung von CNNs oder RNNs in dem Verfahren ermöglicht eine effiziente und genaue Verarbeitung der physiologischen Daten, was zu einer verbesserten Vorhersage der Atemanhaltedauer und einer besseren Anpassung des Messprotokolls führt.

Die Atemanhaltedauer kann eine Atemanhaltedauer nach Exspiration oder eine Atemanhaltedauer nach Inspiration umfassen.

Exspiration ist der Prozess des Ausatmens, während Inspiration der Prozess des Einatmens ist. Die Vorhersage der Atemanhaltedauer nach Exspiration (in einem exspirierten/ausgeatmeten Zustand) und Inspiration (in einem inspirierten/eingeatmeten Zustand) ermöglicht eine genauere Anpassung des Messprotokolls an die individuellen Bedürfnisse der Untersuchungsperson, ohne dass diese beiden Zustände von der Untersuchungsperson testweise eingenommen werden müssen. Durch die Vorhersage von Atemanhaltedauern nach Exspiration und Inspiration kann die Bildqualität weiter verbessert und die Belastung für die Untersuchungsperson reduziert werden.

Die physiologischen Signale können beispielsweise eines oder mehrere umfassen von einem Atemsignal, einem EKG-Signal und einem Pulsoxymeter-Signal.

Ein Atemsignal ist ein Signal, das die Atmung der Untersuchungsperson misst. Ein EKG-Signal ist ein Signal, das die elektrische Aktivität des Herzens misst. Ein Pulsoxymeter-Signal ist ein Signal, das die Sauerstoffsättigung im Blut misst.

Die Verwendung dieser verschiedenen physiologischen Signale ermöglicht eine umfassende und genaue Vorhersage der Atemanhaltedauer, was zu einer verbesserten Bildqualität und einer reduzierten Belastung für die Untersuchungsperson führt.

Die physiologischen Daten können während eines Positionierens der Untersuchungsperson in einem medizinischen Bildgebungssystem oder während einer Justage des medizinischen Bildgebungssystems für das Bildgebungsverfahren erfasst werden.

Das Positionieren der Untersuchungsperson ist der Prozess, bei dem die Untersuchungsperson auf der Liege des medizinischen Bildgebungssystems platziert und innerhalb des medizinischen Bildgebungssystems ausgerichtet wird. Die Justage ist der Prozess der Anpassung und Kalibrierung des medizinischen Bildgebungssystems für die spezifische Untersuchung.

Die Erfassung von physiologischen Daten während des Positionierens oder der Justage ermöglicht eine effiziente Nutzung der Zeit und Ressourcen, ohne zusätzliche Belastung für die Untersuchungsperson oder Verlängerung der Untersuchungsdauer.

Entsprechend dem beschriebenen Verfahren wird im folgenden ein System zur medizinischen Untersuchung einer Untersuchungsperson bereitgestellt.

Ein medizinisches Bildgebungssystem, zum Beispiel eine Magnetresonanztomographie (MRT-) -Anlage oder eine Computertomographie (CT) -Anlage, ist ein technisches System, das dazu dient, Bilder einer Untersuchungsperson für diagnostische oder therapeutische Zwecke zu erstellen.

Ein medizinisches Bildgebungssystem ist ausgebildet, um die folgenden Schritte durchzuführen.

Im Vorfeld einer Datenerfassungsphase des medizinischen Bildgebungsverfahrens werden physiologische Daten einer Untersuchungsperson bereitgestellt. Anschließend wird unter Verwendung der physiologischen Daten eine Atemanhaltedauer während der Datenerfassungsphase des medizinischen Bildgebungsverfahrens für die Untersuchungsperson vorhergesagt. Diese Atemanhaltedauer entspricht der Zeitdauer, während der die Untersuchungsperson ihre Atmung auf Anweisung anhalten soll. Schließlich wird unter Verwendung der vorhergesagten Atemanhaltedauer zumindest ein Parameter eines Messprotokolls konfiguriert, um eine medizinische Bildgebung durchzuführen.

Die Integration des Verfahrens zur Vorhersage der Atemanhaltedauer in das medizinische Bildgebungssystem ermöglicht eine automatisierte und effiziente Anpassung des Messprotokolls, was zu einer verbesserten Bildqualität, einer reduzierten Belastung für die Untersuchungsperson und einer effizienteren Nutzung der Untersuchungszeit führt.

Das medizinisches Bildgebungssystem kann eine Rechenvorrichtung zur Ausführung eines beliebiges Verfahrens oder einer beliebigen Kombination von Verfahren gemäß der vorliegenden Offenbarung enthalten.

Eine Vorrichtung, zum Beispiel ein Rechengerät oder Computer, zum Konfigurieren eines Messprotokolls eines medizinischen Bildgebungsverfahrens umfasst eine Recheneinheit (Prozessor), eine Speichereinheit (Speicher), optional eine Schnittstelle zum Empfangen und Bereitstellen von physiologischen Daten, wobei die Speichereinheit von der Recheneinheit ausführbare Befehle umfasst, die bei der Ausführung durch die Recheneinheit diese veranlassen, die Schritte eines beliebigen Verfahrens oder einer beliebigen Kombination von Verfahren gemäß der vorliegenden Offenbarung auszuführen.

Ein Computerprogramm, oder Computerprogrammprodukt, umfasst Befehle, die bei der Ausführung des Programms durch einen Prozessor diesen veranlassen, die Schritte eines beliebigen Verfahrens oder einer beliebigen Kombination von Verfahren gemäß der vorliegenden Offenbarung auszuführen.

Beispielsweise kann das Computerprogramm Software umfassen, welche man in einen Speicher einer programmierbaren Steuereinrichtung bzw. einer Recheneinheit eines medizinischen Bildgebungssystems laden kann. Mit diesem Computerprogrammprodukt können die beschriebenen Verfahrens ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die beschriebenen Verfahren zu realisieren. Mit anderen Worten soll mit dem auf das Computerprogrammprodukt gerichteten Anspruch insbesondere eine Software unter Schutz gestellt werden, mit welcher die beschriebenen Verfahren ausgeführt werden können. Dabei kann es sich bei der Software um einen Quellcode (z.B. C++), der noch kompiliert und gebunden oder der nur interpretiert werden muss, oder um einen ausführbaren Softwarecode handeln, der zur Aus-führung nur noch in die entsprechende Recheneinheit bzw. Steuereinrichtung zu laden ist.

Ein elektronisch lesbarer Datenträger, umfasst Befehle, die bei der Ausführung durch einen Prozessor diesen veranlassen, die Schritte eines beliebigen Verfahrens oder einer beliebigen Kombination von Verfahren gemäß der vorliegenden Offenbarung auszuführen. Zum Beispiel können die Daten und Befehle zur Ausführung des erfindungsgemäßen Verfahrens in einer verteilten Datenbank, insbesondere einer Cloud, gespeichert sein.

Beispielsweise kann der Datenträger kann eine DVD, ein Magnetband, eine Festplatte oder einen USB-Stick umfassen, auf welchem elektronisch lesbare Steuerinformationen, insbesondere die beschriebene Software, gespeichert ist. Wenn diese Steuerinformationen (Software) von dem Datenträger gelesen und in eine Steuereinrichtung bzw. Recheneinheit einer Magnetresonanzanlage gespeichert werden, können beschriebenen Verfahren durchgeführt werden.

Das medizinische Bildgebungssystem, die Rechenvorrichtung, das Computerprogramm oder Computerprogrammprodukt, und der computerlesbare Datenspeicher sind konfiguriert, um ein beliebiges Verfahren oder eine beliebige Kombination von Verfahren gemäß der vorliegenden Offenbarung durchzuführen.

Für derartige medizinisches Bildgebungssysteme, Vorrichtungen, Computerprogramme, Cloud-Lösungen und elektronisch lesbare Datenträger können technische Effekte erzielt werden, die den technischen Effekten für die Vorrichtung gemäß der vorliegenden Offenbarung entsprechen.

Obwohl die in der obigen Zusammenfassung und der folgenden detaillierten Beschreibung beschriebenen Merkmale im Zusammenhang mit spezifischen Beispielen beschrieben werden, ist zu verstehen, dass die Merkmale nicht nur in den jeweiligen Kombinationen verwendet werden können, sondern auch isoliert oder in beliebigen Kombinationen verwendet werden können, und Merkmale aus verschiedenen Beispielen der Systeme, Vorrichtungen und Verfahren miteinander kombiniert werden können und somit miteinander korrelieren, sofern nicht ausdrücklich anders angegeben.

Die obige Zusammenfassung soll daher nur einen kurzen Überblick über einige Merkmale einiger Ausführungsbeispiele und Implementierungen geben und ist nicht als Einschränkung zu verstehen. Andere Ausführungsformen können weitere als die oben beschriebenen Merkmale umfassen.

### Kurze Beschreibung der Zeichnungen

Die Erfindung wird nachfolgend anhand bevorzugter Ausführungsbeispiele mit Bezug auf die beiliegenden Zeichnungen näher erläutert.

Dabei bezeichnen in den Figuren gleiche Bezugszeichen gleiche oder ähnliche Elemente. Die Figuren sind schematische Darstellungen verschiedener Ausführungsbeispiele der beanspruchten Lösung, wobei die in den Figuren dargestellten Elemente nicht notwendigerweise maßstabsgetreu dargestellt sind. Vielmehr sind die verschiedenen in den Figuren dargestellten Elemente derart wiedergegeben, dass ihre Funktion und genereller Zweck für den Fachmann verständlich wird.
Figur 1 zeigt schematisch eine Magnetresonanzanlage dar, gemäß Ausführungsbeispielen der beanspruchten Lösung.
Figur 2 zeigt schematisch eine Vorhersage von Atemanhaltedauern, gemäß Ausführungsbeispielen der beanspruchten Lösung.
Figur 3 zeigt ein Flussdiagramm mit Schritten eines Verfahrens zum Konfigurieren eines Messprotokolls eines medizinischen Bildgebungsverfahrens, gemäß Ausführungsbeispielen der beanspruchten Lösung.
Figur 4 zeigt schematisch eine Vorrichtung, welche zum Konfigurieren eines Messprotokolls eines medizinischen Bildgebungsverfahrens mittels eines erfindungsgemäßen Verfahrens konfiguriert ist.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung von Ausführungsbeispielen, welche im Zusammenhang mit den Figuren näher erläutert werden.

Dabei ist zu beachten, dass die Beschreibung der Ausführungsbeispiele nicht in einem beschränkenden Sinne zu verstehen ist. Der Umfang der beanspruchten Lösung soll nicht durch die im Folgenden beschriebenen Ausführungsbeispiele oder durch die Figuren eingeschränkt werden, welche nur zur Veranschaulichung dienen.

Nachfolgend werden verschiedene Techniken zum Konfigurieren eines Messprotokolls eines medizinischen Bildgebungsverfahrens detaillierter beschrieben.

Sofern es in der nachfolgenden Beschreibung nicht anders angegeben ist, beziehen sich die Begriffe berechnen, vorhersagen, bestimmen, generieren, konfigurieren, filtern, anpassen und dergleichen vorzugsweise auf Handlungen und/oder Prozesse und/oder Verarbeitungsschritte, die Daten verändern und/oder erzeugen und/oder die Daten in andere Daten überführen, wobei die Daten insbesondere als physikalische Größen dargestellt werden oder vorliegen können, beispielsweise als elektrische Impulse. Insbesondere sollten die Begriffe Computer, Steuergerät oder Vorrichtung möglichst breit ausgelegt werden, um insbesondere alle elektronischen Vorrichtungen mit Datenverarbeitungseigenschaften abzudecken. Computer können somit beispielsweise Personal Computer, Server, speicherprogrammierbare Steuerungen (SPS), Handheld-Computer-Systeme, Pocket-PC-Vorrichtungen, IoT-Vorrichtungen Mobilfunkgeräte und andere Kommunikationsgeräte, Cloud-Applikationen, Prozessoren und andere elektronische Vorrichtungen zur Datenverarbeitung sein, d.h. die rechnergestützt Daten verarbeiten können.

### Detaillierte Beschreibung von Ausführungsbeispielen

Viele MR-Untersuchungen, insbesondere im abdominellen Bereich, machen eine Aufnahme unter Atemanhalten erforderlich, um Bildartefakte zu vermeiden. Dabei wird die Aufnahme in der Regel in mehrere Teilabschnitte unterteilt, die jeweils von Atemkommandos unterbrochen werden.

Eine Herausforderung besteht darin, die Atemanhalte-Fähigkeit der Untersuchungsperson bereits vor dem Start der Bildgebung richtig vorherzusagen. Eine überschätzte Atemanhalte-Dauer führt dabei zu verstärkten Bildartefakten, wenn die Untersuchungsperson abrupt einatmen muss, während eine unterschätzte Dauer in unnötigen zusätzlichen Teilabschnitten und damit längerer Messdauer resultiert.

Es wird daher ein erweitertes Verfahren vorgeschlagen, was ohne zusätzlichen Probescan auskommt und die Atemanhaltedauer indirekt aus anderen Parametern vorhersagen soll. Es liegt der Annahme zugrunde, dass die Atemanhaltekapazität durch Faktoren wie Lungenvolumen und Sauerstoffsättigung des Blutes gegeben ist aber auch z.B. die Form der regulären Atemkurve Rückschlüsse auf die Kapazität zulässt. Die Daten sollen dabei ohne Zusatzaufwand ermittelt werden, z.B. in den Minuten der Untersuchung vor der Atemanhalte-Messung, d.h. während der Positionierung der Untersuchungsperson auf der Liege, der Justage-Phase oder vorausgehenden Scans ohne Atemkommandos.

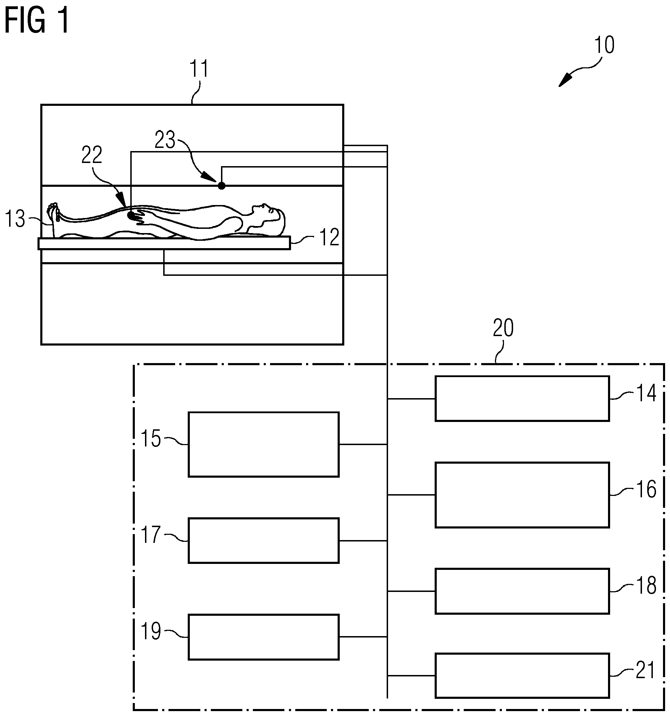

Figur 1 zeigt schematisch eine Magnetresonanzanlage 10 dar, gemäß Ausführungsbeispielen der beanspruchten Lösung.

Wie in Fig. 1 zu sehen, wird eine Magnetresonanzanlage 10 dargestellt, mit der, wie nachfolgend erläutert wird, erfindungsgemäß ein Messprotokoll generiert, angepasst und durchgeführt werden kann. Die Magnetresonanzanlage 10 weist einen Magneten 11 zur Erzeugung eines Polarisationsfelds B0 auf, wobei eine auf einer Liege 12 angeordnete Untersuchungsperson 13, d.h. ein Patient oder eine Patientin 13 in den Magneten 11 gefahren wird, um dort ortskodierte Magnetresonanzsignale bzw. MR-Daten aus der Untersuchungsperson 13 aufzunehmen. Die zur Signalaufnahme verwendeten Spulen wie eine Ganzkörperspule oder Lokalspulen sind aus Übersichtlichkeitsgründen nicht dargestellt. Durch Einstrahlen von Hochfrequenzpulsen und Schalten von Magnetfeldgradienten kann die durch das Polarisationsfeld B0 erzeugte Magnetisierung aus der Gleichgewichtslage ausgelenkt und ortskodiert werden, und die sich ergebende Magnetisierung wird von den Empfangsspulen detektiert. Wie durch Einstrahlen der HF-Pulse und durch Schalten von Magnetfeldgradienten in verschiedenen Kombinationen und Reihenfolgen MR-Bilder erzeugt werden können, ist dem Fachmann grundsätzlich bekannt und wird hier nicht näher erläutert.

In der Magnetresonanzanlage 10 sind weiterhin Sensoren 22, 23 angeordnet, welche physiologische Signale der Untersuchungsperson 13 überwachen. Ein erster Sensor 22 ist an einer Hand der Untersuchungsperson 13 angeordnet, ein zweiter Sensor 23 in der Nähe des Brustkorbs der Untersuchungsperson. Es ist möglich, dass die Sensoren in Kontakt und/oder entfernt von der Untersuchungsperson ohne direkten Kontakt, im Innenraum oder beispielweise in der Liege 12 angeordnet sind. Die Messsignale der Sensoren 22, 23 werden über drahtgebundene oder drahtlose Übertragung einer Rechenvorrichtung oder Steuereinheit 20 der Magnetresonanzanlage 10 bereitgestellt. Weiter ist zu verstehen, dass die Anzahl und Art von Sensoren eine beliebige sein kann.

Die physiologischen Sensoren 22, 23 können kontinuierlich, oder in vorbestimmten Zeitintervallen, physiologische Signale (Daten) der Untersuchungsperson erfassen. Insbesondere kann die Untersuchungsperson durch die Sensoren bereits vor Beginn des bildgebenden Verfahrens überwacht werden, d.h. beispielsweise beim Liegen auf der Liege beim Einfahren, bei schnellen Vorbereitungsscans ohne Atemanweisungen oder bei Justagevorgängen des Bildgebungssystems.

Die Magnetresonanzanlage 10 weist weiterhin eine Steuereinheit 20 auf, die zur Steuerung der Magnetresonanzanlage 10 verwendet werden kann. Die Steuerung 20 weist eine Gradientensteuereinheit 15 zur Steuerung und Schaltung der notwendigen Magnetfeldgradienten auf. Eine HF-Steuereinheit 14 ist für die Steuerung und Generierung der HF-Pulse zur Auslenkung der Magnetisierung vorgesehen. Ei-ne Bildsequenzsteuerung 16 steuert die Abfolge der Magnetfeldgradienten und HF-Pulse und damit indirekt die Gradientensteuereinheit 15 und die HF-Steuereinheit 14. Über eine Eingabeeinheit 17 kann eine Bedienperson die Magnetresonanzanlage 10 steuern, und auf einer Anzeigeeinheit 18 können MR-Bilder und sonstige zur Steuerung notwendigen Informationen angezeigt werden. Eine Recheneinheit 19 mit mindestens einer Prozessoreinheit (nicht gezeigt) ist vor-gesehen zur Steuerung der verschiedenen Einheiten in der Steuereinheit 20 und zur Durchführung von Rechenoperationen. Beispielweise könnte zusätzlich eine Kamera (nicht abgebildet) vorgesehen sein, mit welcher Bilder der Untersuchungsperson 13 erfasst werden können. Die Recheneinheit 19 ist ausgebildet, um aus den erfassten MR-Signalen die MR-Bilder zu berechnen.

Während einer Untersuchung wird die Untersuchungsperson 13 aufgefordert, ihren Atem nach Exspiration oder Inspiration anzuhalten, um möglichst keine Bewegung durchzuführen, die das bildgebende Messverfahren stört.

Die Magnetresonanzanlage 10 ist ausgebildet, um ein erfindungsgemäßes Verfahren zum Konfigurieren eines Messprotokolls eines medizinischen Bildgebungsverfahrens auszuführen, und weiterhin eine Bildgebung basierend auf dem Messprotokoll auszuführen.

Das Verfahren umfasst ein Bereitstellen von physiologischen Daten der Untersuchungsperson 13 im Vorfeld einer Datenerfassungsphase des medizinischen Bildgebungsverfahrens. Dabei werden kontinuierlich physiologische Signale 2 der Untersuchungsperson 13 erfasst, beispielsweise mittels Sensoren 22 und 23, die an oder in der Nähe der Untersuchungsperson angeordnet sind. Die Erfassung der physiologischen Signale 2 kann in einer Präparationsphase des medizinischen Bildgebungsverfahrens erfolgen, während der Positionierung der Untersuchungsperson auf der Liege, der Justage-Phase oder vorausgehenden Scans ohne Atemkommandos. Beispielsweise können die physiologischen Signale ein Atemsignal, ein EKG-Signal und/oder ein Pulsoxymeter-Signal umfassen. Die physiologischen Daten 1, die zur Vorhersage verwendet werden, können auch aus einer Patientenakte, Messwerten oder Rohdaten von vorhergehenden Untersuchungen an der Untersuchungsperson stammen.

Zum Beispiel können Informationen zur Sauerstoffsättigung aus einem HIS/RIS-System oder einer angebundenen Smartwatch/Wearable vorliegen und in den physiologischen Daten 1 enthalten sein.

Ein HIS-System ist ein Krankenhausinformationssystem (HIS, engl. Hospital Information System), ein RIS-System ist eine Radiologieinformationssystem (RIS, engl. Radiology Information System). Ein HIS ist ein computergestütztes System, das dazu dient, den Informationsfluss innerhalb eines Krankenhauses oder einer Klinik zu organisieren und zu verwalten. Ein RIS hingegen ist ein spezielles Informationssystem, das sich auf die Verwaltung von radiologischen Informationen und Prozessen konzentriert. Beide Systeme arbeiten oft zusammen, um den Informationsaustausch zwischen verschiedenen Abteilungen und Fachbereichen zu erleichtern und die Effizienz der medizinischen Versorgung zu verbessern.

In einem weiteren Beispiel kann das Lungenvolumen aus im Rahmen der Untersuchung ohnehin durchgeführten Aufnahmen, wie Justage- oder Localizer-Aufnahmen, geschätzt werden.

Das Verfahren kann weiterhin so gestaltet sein, dass während des kontinuierlichen Erfassens der physiologischen Signale 2 der Untersuchungsperson 13 keine Anweisung zum Anhalten der Atmung bereitgestellt wird.

Im Beispiel des MRT-Systems 10 können die physiologischen Signale 2 unter Verwendung eines Pilot-Tone-Messverfahrens in einem Frequenzbereich erfasst werden, der außerhalb des für die Erfassung von Bilddaten verwendeten Frequenzbereichs liegt. Die Verwendung von mit dem Pilot Tone-Verfahren gewonnenen Signalen ist besonders vorteilhaft, da dieses Verfahren sowohl für Herzschlag als auch Lungenvolumenveränderungen sensitiv ist.

Beispielsweise kann das Lungenvolumen aus im Rahmen der Untersuchung ohnehin durchgeführten Aufnahmen, wie Justage- oder Localizer-Aufnahmen, geschätzt werden. Hierfür bietet sich ein separat zu trainierendes Netzwerk an. Alternativ können die Bilddaten als zusätzliche Eingangskanäle des ResNet-Netzes hinzugefügt werden. Bei dem Verfahren zur Schätzung des Lungenvolumens aus Justage- oder Localizer-Daten ist zum Training eine akkurate Bestimmung des Lungenvolumens mit einer passenden Bildgebungssequenz, wie beispielsweise einer VIBE-Sequenz, erforderlich.

Justage- und Localizer-Aufnahmen sind spezielle Arten von Bildern, die in der Magnetresonanztomographie (MRT) erstellt werden, um die Untersuchungsperson und das MRT-System für die eigentliche Bildgebung vorzubereiten.

Justage-Aufnahmen dienen der Kalibrierung und Anpassung des MRT-Systems, um Bildqualität und Signalintensität zu gewährleisten. Sie werden vor der eigentlichen Untersuchung durchgeführt und ermöglichen die Anpassung der Magnetfeldhomogenität, der Empfangsspulenanordnung und der Sendeleistung des Hochfrequenzpulses.

Localizer-Aufnahmen, auch bekannt als Orientierungsaufnahmen oder Scout-Aufnahmen, werden zu Beginn der MRT-Untersuchung erstellt, um den Untersuchungsbereich und die genaue Position der Untersuchungsperson innerhalb der MRT-Gerätschaft zu bestimmen.

Die VIBE-Sequenz (Volumetric Interpolated Breathhold Examination) ist eine MRT-Bildgebungssequenz mit schneller Datenerfassung und hohe räumliche Auflösung, mit deren Hilfe das Lungenvolumen einer Untersuchungsperson bestimmt werden kann.

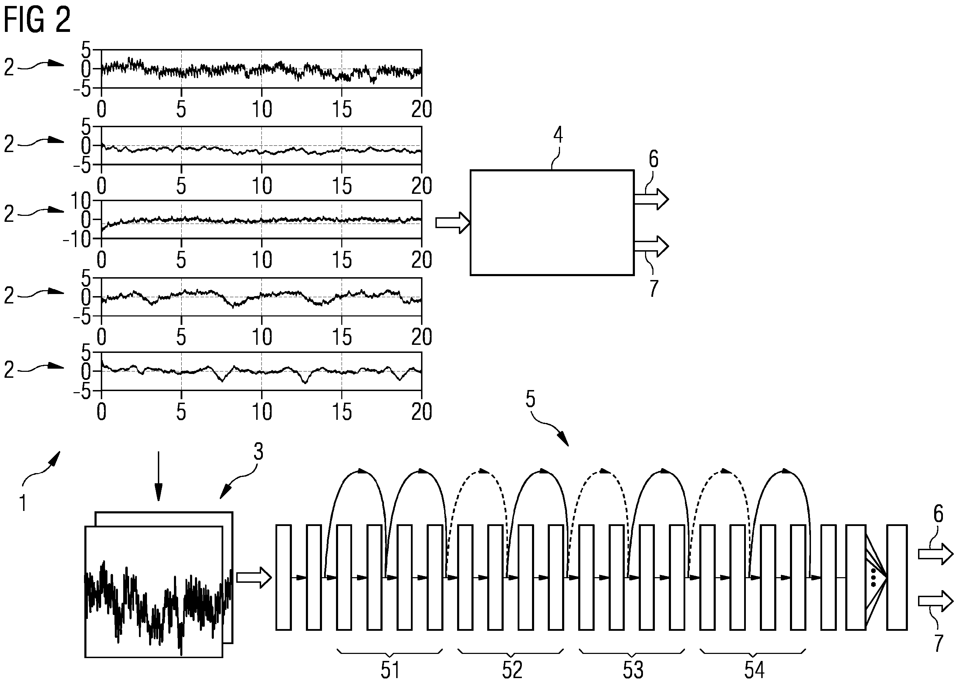

Basierend auf diesen physiologischen Daten, wird einer Atemanhaltedauer, welche von der Untersuchungsperson während der Datenerfassungsphase des medizinischen Bildgebungsverfahrens einzuhalten ist, bestimmt. Dabei entspricht die Atemanhaltedauer einer Zeitdauer, während der die Untersuchungsperson ihre Atmung auf Anweisung anhalten soll. Die Atemanhaltedauer wird unter Verwendung der physiologischen Daten 1 vorhergesagt, wobei beispielsweise ein neuronale Netzwerk eingesetzt werden kann, wie in Figur 2 genauer beschrieben.

Schließlich wird zumindest ein Parameter des Messprotokolls unter Verwendung der vorhergesagten Atemanhaltedauer konfiguriert.

Somit ermöglicht das erfindungsgemäße Verfahren für MRT-Anlage 10 eine verbesserte Vorhersage der Atemanhaltedauer der Untersuchungsperson 13 ohne einen zusätzlichen Probescan. Dabei wird die Atemanhaltekapazität indirekt beispielsweise aus anderen Parametern, wie Lungenvolumen und Sauerstoffsättigung des Blutes, vorhergesagt. Durch die Anwendung dieses Verfahrens werden Vorteile wie keine Verlängerung der Scanzeit, Schonung der Untersuchungsperson, Vorhersage von Dauern, die über die Dauer des Probescans hinausgehen, und Vorhersage von Dauern nach Inspiration und Exspiration erzielt. Das erfinderische Verfahren ermöglicht somit eine optimierte und patientenschonende Durchführung von MR-Untersuchungen, insbesondere im abdominellen Bereich.

Figur 2 zeigt schematisch eine Vorhersage von Atemanhaltedauern 6,7, gemäß Ausführungsbeispielen der beanspruchten Lösung.

Zur Vorhersage von Atemanhaltedauern basierend auf physiologischen Daten 1, insbesondere physiologischen Signalen 2 einer Untersuchungsperson, kann beispielsweise eins von zwei alternative neuronale Netzwerke 4,5 verwendet werden.

In einem Ausführungsbeispiel kann ein neuronales Netz trainiert werden, welches die kontinuierlich aufgezeichneten Kurven von physiologischen Sensoren, wie Sensoren 22 und 23, als Eingangsdaten erhält. Die physiologischen Daten 1 könnten insbesondere ein Atemsignal, die EKG-Signale und ein Pulsoxymeter-Signal umfassen.

Das trainierte neuronale Netzwerk soll die geschätzte Atemanhalte-Zeit nach Inspiration bzw. Exspiration vorhersagen. Als Netzarchitektur bietet sich hier ein Recurrent Neural Network (RNN), beispielsweise in Form eines Long Short Term Memory (LSTM) Netzwerk 4, an. Die physiologischen Signale 2 bzw. können in definierte Abschnitte der Signale, wie etwa 30 Sekunden, in grafische Form, i.e. Bilder 3, überführt werden. Dies kann auch eine geeignete Transformation, z.B. in Polarkoordinaten mittels Gramian Angular Field-Methode, umfassen. Hierbei kann jedes Signal in einen Farbkanal überführt werden und als Eingabebilder 3 für ein auf der ResNet-Architektur 5 basierendes Netz dienen.

Alle Verfahren erfordern passend annotierte Trainingsdaten. Entsprechende Signalkurven lassen sich leicht sammeln, da die physiologischen Signale seit vielen Jahren zusammen mit den aufgezeichneten Rohdaten abgespeichert werden. Daher können die Signale von Untersuchungen, die Protokolle mit Atemkommandos enthalten, automatisiert ermittelt und die physiologischen Signale 2 extrahiert werden. Jedoch ist ein manuelles Annotieren (Labelling) der exakten Atemanhalte-Dauer erforderlich, zum Beispiel durch Ermittlung des Start- und Endzeitpunkts der entsprechenden Protokolle und anschließendem Auswählen der Zeitpunkte von Start und Ende des Atemanhaltens.

Möglich ist auch eine dedizierte Aufnahme mit einem Kollektiv an Probanden, bei die Probanden zunächst still liegen, um ei-ne Zeitlang Signale zu sammeln und anschließend Kommandos zum möglichst langem Atemanhalten nach Inspiration und Exspiration gegeben werden. Bei der Vorhersage der Atemanhaltedauer werden die Atemanhaltedauer 6 für Inspiration und Atemanhaltedauer 7 für Exspiration berücksichtigt. Das neuronale Netzwerk 4, beispielsweise ein LSTM-Netz, oder das neuronale Netzwerk 5, basierend auf der ResNet-Architektur, dient zur Vorhersage dieser Dauern, wie nachfolgende in Bezug auf Figur 2 beschrieben wird.

Im oberen Teil der Figur 2 ist ein neuronales Netzwerk mit LSTM-Architektur 4 zur Verarbeitung und Analyse physiologischer Daten 1, insbesondere der physiologischen Signale 2, dargestellt.

Die LSTM-Architektur 4 (Long Short-Term Memory) ist eine spezielle Form von rekurrenten neuronalen Netzwerken (RNN), die sich besonders gut für die Verarbeitung von sequentiellen Daten eignet. Im Gegensatz zu herkömmlichen RNNs verfügen LSTM-Netzwerke über eine zusätzliche Speicherzelle, die es ihnen ermöglicht, langfristige Abhängigkeiten zwischen Datenpunkten besser zu erkennen und zu lernen. Dies macht sie besonders geeignet für Anwendungen, bei denen zeitabhängige Muster von Bedeutung sind, wie beispielsweise bei der Vorhersage der Atemanhaltefähigkeit 6,7 basierend auf physiologischen Signalen 2.

Als Eingabe in das neuronale Netzwerk 4 werden die physiologischen Signale 2 bereitgestellt, wie beispielsweise innerhalb eines vorbestimmten Zeitintervalls aufgezeichnet wurden. Als Ausgabe stellt das neuronale Netzwerk zwei Atemanhaltedauern bereit, eine Atemanhaltedauer nach Inspiration 6 und eine Atemanhaltedauer nach Exspiration 7.

Im unteren Teil der Figur 2 ist ein neuronales Netzwerk mit Resnet18-Architektur 5 zur Verarbeitung und Analyse physiologischer Daten 1, insbesondere der in Bilddaten 3 konvertierten Signale 2, dargestellt.

Die ResNet-Architektur 5 (Residual Network) ist eine Form von tiefen neuronalen Netzwerken, die sich durch ihre Fähigkeit auszeichnen, tiefe Netzwerkstrukturen effektiv zu trainieren. ResNet erreicht dies durch die Einführung von sogenannten "Residual Blocks", die es dem Netzwerk ermöglichen, Informationen über viele Schichten hinweg effizient zu übertragen. ResNet-Architekturen haben sich in verschiedenen Bildverarbeitungsaufgaben als sehr leistungsfähig erwiesen und könnten auch zur Analyse von physiologischen Signalen in grafischer Form verwendet werden, um die Atemanhaltefähigkeit vorherzusagen.

Die Resnet-Architektur 5 umfasst vier Schichten 51, 52, 53, 54, die jeweils aus identischen ConvNet-Schichten bestehen. In jeder Schicht 51, 52, 53, 54, erhöht sich die Anzahl der Filter, während die Eingabegröße verringert wird. Die Architektur ist optimiert, indem Verbindungen zwischen den ConvNets übersprungen werden. Nach den Hauptblöcken wird eine Average-Pooling-Schicht und zwei vollständig verbundene Schichten angewendet.

Die Ausgabe des Netzes sind zwei vorhergesagte Atemanhaltedauern für Inspiration 6 und Exspiration 7, basierend auf den analysierten physiologischen Daten 3.

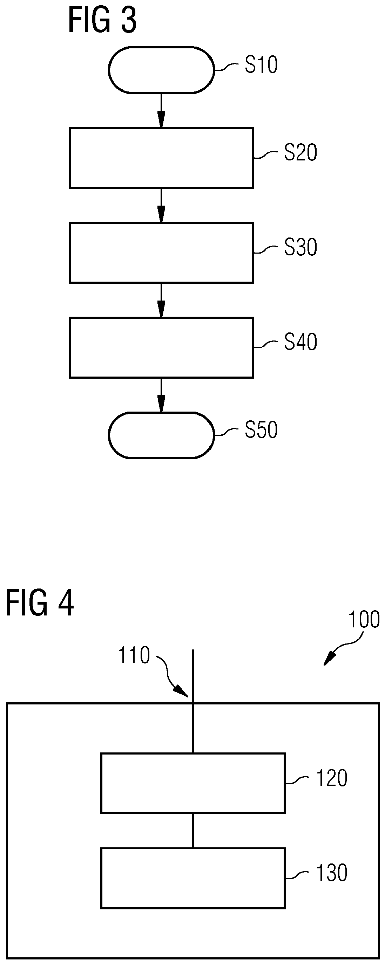

Figur 3 zeigt ein Flussdiagramm mit Schritten eines Verfahrens zum Konfigurieren eines Messprotokolls eines medizinischen Bildgebungsverfahrens, gemäß Ausführungsbeispielen der beanspruchten Lösung.

Das Verfahren beginnt in Schritt S10. In Schritt S20, im Vorfeld einer Datenerfassungsphase des medizinischen Bildgebungsverfahrens, wird das Bereitstellen von physiologischen Daten einer Untersuchungsperson durchgeführt. In Schritt S30 erfolgt das Vorhersagen, unter Verwenden der physiologischen Daten, einer Atemanhaltedauer während einer Datenerfassungsphase des medizinischen Bildgebungsverfahrens für die Untersuchungsperson, welche einer Zeitdauer entspricht, während der die Untersuchungsperson ihre Atmung auf Anweisung anhalten soll. In Schritt S40 wird das Konfigurieren, unter Verwenden der vorhergesagten Atemanhaltedauer, zumindest eines Parameters des Messprotokolls durchgeführt. Das Verfahren endet in Schritt S50.

Figur 4 zeigt schematisch eine Vorrichtung 100, welche zum Konfigurieren eines Messprotokolls eines medizinischen Bildgebungsverfahrens mittels eines erfindungsgemäßen Verfahrens konfiguriert ist.

Die Vorrichtung 100, umfasst einen Prozessor 130, einen Speicher 120, und eine Schnittstelle 110 zum Empfangen von Daten, wobei der Speicher 120 von dem Prozessor 130 ausführbare Befehle umfasst, die bei der Ausführung durch den Prozessor 130 die Vorrichtung 100 veranlassen, die Schritte eines beliebigen Verfahrens oder einer beliebigen Kombination von Verfahren gemäß der vorliegenden Offenbarung auszuführen.

Aus dem oben Gesagten lassen sich einige allgemeine Schlussfolgerungen ziehen:
In einigen Beispielen können physiologische Daten als Informationen über den Zustand oder die Funktion des Körpers der Untersuchungsperson verstanden werden, die zur Vorhersage der Atemanhaltedauer während eines medizinischen Bildgebungsverfahrens verwendet werden können. Diese Daten können aus verschiedenen Quellen stammen und unterschiedliche Aspekte der Physiologie der Untersuchungsperson abbilden.

Im Allgemeinen können maschinelles Lernen, Deep Learning und künstliche neuronale Netzwerke verwendet werden, um die Atemanhaltedauer einer Untersuchungsperson zu bestimmen. Maschinelles Lernen ist ein Teilbereich der künstlichen Intelligenz und umfasst verschiedene Algorithmen und Techniken, um Muster in Daten zu erkennen und Vorhersagen zu treffen. ML-Ansätze können für die Vorhersage der Atemanhaltefähigkeit nützlich sein, insbesondere wenn geeignete Merkmale (Features) aus den physiologischen Signalen extrahiert werden. Allerdings können traditionelle ML-Verfahren in einigen Fällen Schwierigkeiten haben, komplexe Muster in den Daten zu erkennen.

Deep Learning ist eine Untergruppe des maschinellen Lernens und bezieht sich auf den Einsatz von künstlichen neuronalen Netzwerken mit vielen Schichten (tiefen Netzwerken). Deep Learning hat in vielen Anwendungen beeindruckende Ergebnisse erzielt, besonders bei der Verarbeitung von Bildern, Sprache und Zeitreihen. Bei der Vorhersage der Atemanhaltefähigkeit könnte Deep Learning dazu beitragen, sowohl die Merkmalsextraktion als auch die Modellierung komplexer Zusammenhänge automatisch durchzuführen, was möglicherweise zu genaueren Vorhersagen führt.

Künstliche neuronale Netzwerke umfassen sowohl traditionelle neuronale Netzwerke als auch tiefere Architekturen wie LSTM und ResNet, die zuvor besprochen wurden. Neuronale Netzwerke sind sehr flexibel und können für eine Vielzahl von Aufgaben eingesetzt werden, einschließlich der Vorhersage der Atemanhaltefähigkeit (Atemanhaltedauer).

Insgesamt hängt die Leistung jeder dieser Techniken von der spezifischen Anwendung, den verfügbaren Daten und der Komplexität der zu modellierenden Zusammenhänge ab. Die Wahl der besten Methode kann von Fall zu Fall variieren, und es kann sinnvoll sein, mehrere Ansätze in Kombination oder im Rahmen von Ensemble-Methoden zu verwenden, um die Vorhersagegenauigkeit weiter zu verbessern.

Neuronale Netzwerke sind eine Art von maschinellen Lernverfahren, die auf der Struktur und Funktionsweise des menschlichen Gehirns basieren. Sie bestehen aus einer Vielzahl von künstlichen Neuronen, die miteinander verbunden sind und Informationen verarbeiten können. Neuronale Netzwerke können für eine Vielzahl von Aufgaben eingesetzt werden, darunter Mustererkennung, Klassifikation, Regression und Vorhersage.

Ein neuronales Netzwerk besteht aus mehreren Schichten, wobei die erste Schicht die Eingabeschicht ist, die die Eingangsdaten empfängt, und die letzte Schicht die Ausgabeschicht ist, die die Ergebnisse liefert. Dazwischen liegen eine oder mehrere verborgene Schichten, die für die Verarbeitung und das Lernen der Daten verantwortlich sind. Neuronale Netzwerke werden durch einen Trainingsprozess "trainiert", bei dem sie anhand von Beispieldaten angepasst werden, um die gewünschten Ergebnisse zu erzielen.

Ein konkretes Beispiel für die Anwendung eines neuronalen Netzwerks ist die Vorhersage der Atemanhaltefähigkeit basierend auf physiologischen Daten oder Signalen. In diesem Fall könnten Eingangsdaten wie Atemsignal, EKG-Signale und Pulsoxymeter-Signale verwendet werden. Das neuronale Netzwerk kann darauf trainiert werden, aus diesen Daten die Atemanhaltedauer, in anderen Worten Atemanhaltezeit, oder -zeitdauer, nach Inspiration bzw. Exspiration vorherzusagen.

Dafür kann beispielsweise ein rekurrentes neuronales Netzwerk (RNN) in Form eines Long-Short-Term-Memory (LSTM)-Netzes verwendet werden, das besonders gut für die Verarbeitung von zeitabhängigen Daten geeignet ist. Alternativ könnten die physiologischen Signale in grafische Form überführt und als Eingabebilder für ein auf der ResNet-Architektur basierendes Netz verwendet werden.

Neben LSTM und ResNet gibt es noch andere mögliche neuronale Netzwerkarchitekturen, die für die Vorhersage der Atemanhaltedauer in Betracht gezogen werden könnten. Dazu gehören beispielsweise Convolutional Neural Networks (CNNs), die sich besonders gut für die Verarbeitung von bild- und signalbasierten Daten eignen, oder auch Attention-basierte Modelle, die in der Lage sind, auf relevante Informationen innerhalb der Eingangsdaten zu fokussieren.

Durch das Training des neuronalen Netzwerks auf passend annotierten Trainingsdaten kann das Netzwerk schließlich in der Lage sein, die Atemanhaltefähigkeit einer Untersuchungsperson vorherzusagen, ohne dass ein dedizierter Vorscan durchgeführt werden muss. Dies bietet Vorteile wie eine kürzere Messzeit, Schonung der Untersuchungsperson und die Vorhersage von Atemanhaltezeiten nach Inspiration und Exspiration.

Alternativ zur Verwendung eines neuronalen Netzes gibt es verschiedene alternative Ansätze zur Vorhersage der Atemanhaltedauer aus physiologischen Signalen, die von regelbasierten Algorithmen über statistische Modelle und maschinelles Lernen bis hin zu Expertensystemen reichen.

Beispielsweise könnte eine Methode zur Vorhersage der Atemanhaltedauer aus physiologischen Signalen auf regelbasierten Algorithmen basieren. In diesem Ansatz würden bestimmte Muster und Merkmale in den physiologischen Daten identifiziert, die auf die Atemanhaltekapazität der Untersuchungsperson hindeuten. Diese Merkmale könnten beispielsweise die Atemfrequenz, die Amplitude der Atembewegungen oder die Herzfrequenz umfassen. Durch die Analyse dieser Merkmale und den Vergleich mit vordefinierten Schwellenwerten oder Regeln könnte die Atemanhaltedauer geschätzt werden.

Eine weitere Möglichkeit wäre die Anwendung von statistischen Modellen oder maschinellem Lernen, das nicht auf neuronalen Netzen basiert. Beispielsweise könnten lineare oder nichtlineare Regressionsmodelle verwendet werden, um Zusammenhänge zwischen den physiologischen Daten und der Atemanhaltedauer herzustellen. In diesem Fall würden die Modelle anhand von Trainingsdaten erstellt und optimiert, um Vorhersagen über die Atemanhaltekapazität zu treffen.

Schließlich könnten auch Expertensysteme eingesetzt werden, die auf dem Wissen von Fachleuten aus dem Bereich der medizinischen Bildgebung basieren. Solche Systeme würden menschenähnliche Entscheidungsprozesse nachahmen, indem sie auf einer Wissensbasis aufbauen, die aus Erfahrungen und Erkenntnissen von Experten stammt. In diesem Szenario könnten die Expertensysteme physiologische Daten analysieren und unter Berücksichtigung von Expertenwissen Empfehlungen für die optimale Atemanhaltedauer geben.

In einigen Beispielen können physiologische Daten Informationen über das Alter, das Geschlecht, das Gewicht, die Größe und den allgemeinen Gesundheitszustand der Untersuchungsperson umfassen. Diese Informationen können verwendet werden, um Rückschlüsse auf die Fähigkeit der Untersuchungsperson zu ziehen, ihre Atmung für einen bestimmten Zeitraum anzuhalten. Diese Daten können beispielsweise aus einer Patientenakte, oder einer elektronischen Datenbank stammen.

In einigen Beispielen können physiologische Daten Daten über die Lungenfunktion der Untersuchungsperson enthalten, wie beispielsweise die das Lungenvolumen, die Lungenkapazität (Vitalkapazität), das forcierte Exspirationsvolumen, oder das funktionelle Residualvolumen. Diese Lungenfunktionsparameter können dazu beitragen, die Atemanhaltedauer der Untersuchungsperson genauer vorherzusagen.

In weiteren Beispielen kann die Herzfrequenz der Untersuchungsperson als physiologischer Parameter verwendet werden, um die Atemanhaltedauer vorherzusagen. Eine erhöhte Herzfrequenz kann auf eine eingeschränkte Atemfunktion oder eine allgemein verringerte Fähigkeit zur Atemanhaltung hindeuten.

Zum Beispiel können physiologische Daten auch Informationen über die körperliche Fitness der Untersuchungsperson beinhalten, wie zum Beispiel den Body-Mass-Index (BMI), den maximalen Sauerstoffverbrauch (V02max) oder den Trainingszustand der Untersuchungsperson. Diese Informationen können helfen, die Atemanhaltedauer besser einzuschätzen, da Patienten mit einer höheren körperlichen Fitness in der Regel eine längere Atemanhaltedauer haben.

In einigen Beispielen können physiologische Daten auch aus der direkten Messung der Atemfrequenz und -tiefe (Atemkurve) der Untersuchungsperson stammen. Diese Messungen können durchgeführt werden, bevor das medizinische Bildgebungsverfahren beginnt, und können verwendet werden, um eine individualisierte Vorhersage der Atemanhaltedauer für die Untersuchungsperson zu ermöglichen.

In einigen Beispielen bezieht sich die Atemanhaltedauer auf den Zeitraum, während dem eine Untersuchungsperson ihre Atmung auf Anweisung während des medizinischen Bildgebungsverfahrens anhalten kann. Die Atemanhaltedauer kann variieren, abhängig von den individuellen physiologischen Eigenschaften der Untersuchungsperson und der jeweiligen Bildgebungstechnik und deren Einflüsse auf die Physis und Psyche der Patienten.

In einigen Beispielen kann die Atemanhaltedauer als die Zeit definiert werden, die eine Untersuchungsperson ohne unangenehme Empfindungen oder körperliche Belastung die Atmung anhalten kann. Diese Atemanhaltedauer kann dazu beitragen, die Qualität der aufgenommenen Bilder zu verbessern, indem Bewegungsartefakte reduziert werden.

In einigen Beispielen kann die Atemanhaltedauer in Zusammenhang mit spezifischen medizinischen Bildgebungsverfahren stehen, wie beispielsweise der Magnetresonanztomographie (MRT) oder der Computertomographie (CT). Ähnliche Techniken können ohne weiteres auf verschiedene andere Arten und Typen digitaler Bildgebungssysteme angewandt werden, z. B. Röntgen- und Ultraschall-Bildgebungssysteme (Sonographie), die eine Vielzahl von Bildern aus Messdaten eines Untersuchungsobjekts oder Patienten generieren können. In solchen Fällen kann die Atemanhaltedauer als die Zeit definiert werden, die eine Untersuchungsperson ihre Atmung anhalten muss, um eine vollständige Bildsequenz oder einen bestimmten Schritt im Bildgebungsverfahren abzuschließen.

Zum Beispiel kann die Atemanhaltedauer auch als eine Funktion der Bildqualität betrachtet werden. Hierbei wird die Atemanhaltedauer so gewählt, dass sie den Kompromiss zwischen der erforderlichen Bildqualität und dem Komfort der Untersuchungsperson optimiert. Dies kann bedeuten, dass die Atemanhaltedauer kürzer sein kann, wenn eine geringere Bildqualität für die Diagnose oder Behandlung ausreichend ist, oder länger, wenn eine höhere Bildqualität erforderlich ist.

In einigen Beispielen kann die Atemanhaltedauer auch im Zusammenhang mit der Atmungssynchronisation bei medizinischen Bildgebungsverfahren definiert werden. In solchen Fällen kann die Atemanhaltedauer als der Zeitraum betrachtet werden, in dem die Untersuchungsperson ihre Atmung anhalten kann, um die Bildgebung während einer bestimmten Phase des Atemzyklus durchzuführen, um so optimale Bildqualität zu gewährleisten.

Beispielsweise kann eine vorhergesagt Atemanhaltedauer einen Zeitraum von >1 Sekunde, >10 Sekunden, >20 Sekunden oder >30 Sekunden umfassen, währenddessen die Untersuchungsperson keine Atmungsaktivität /-bewegung ausführt.

Im Allgemeinen kann bei der Atemanhaltedauer auch von einer Atemhaltefähigkeit, oder -kapazität der Untersuchungsperson gesprochen werden, welche durch einen oder mehrere Kennzahlen repräsentiert sein kann.

In einigen Beispielen kann sich ein Messprotokoll auf die spezifischen Schritte und Einstellungen, die in einem medizinischen Bildgebungsverfahren verwendet werden, um die gewünschten Bilder der Untersuchungsperson zu erhalten, beziehen. Das Messprotokoll kann je nach der Art des Bildgebungsverfahrens und den individuellen Anforderungen der Untersuchungsperson variieren.

In einigen Beispielen kann ein Messprotokoll als eine Reihe von Anweisungen oder Einstellungen definiert werden, die den Betrieb und die Steuerung des medizinischen Bildgebungssystems während des gesamten Bildgebungsverfahrens bestimmen. Dies kann beispielsweise die Wahl der Bildgebungstechnik, die Sequenzierung der Aufnahmen, die räumliche Auflösung und die Bildkontrasteinstellungen beinhalten.

In einigen Beispielen kann ein Messprotokoll speziell auf ein bestimmtes medizinisches Bildgebungsverfahren wie Magnetresonanztomographie (MRT), Computertomographie (CT) oder Positronenemissionstomographie (PET) zugeschnitten sein. In solchen Fällen können die spezifischen Schritte und Einstellungen des Messprotokolls von der jeweiligen Technik abhängig sein und beispielsweise die Einstellung der Magnetfeldstärke, die Wahl der Schichtdicke oder die Verwendung von Kontrastmitteln umfassen.

In einigen Beispielen beziehen sich Parameter auf variable Werte des Messprotokolls, die angepasst oder konfiguriert werden können, um die Durchführung des medizinischen Bildgebungsverfahrens zu ermöglichen, zu steuern, sowie insbesondere, um die Bildqualität und den Komfort der Untersuchungsperson während des medizinischen Bildgebungsverfahrens zu optimieren.

In einigen Beispielen können Parameter die zeitliche und räumliche Auflösung der Bilder, die Intensität der Magnetfelder oder Röntgenstrahlen, die Anzahl der aufgenommenen Bilder, die verwendete Kontrastmittelmenge oder die Dauer der Bildaufnahme beeinflussen. Die Anpassung dieser Parameter kann dazu beitragen, das Bildgebungsverfahren an die spezifischen Bedürfnisse und Eigenschaften der Untersuchungsperson anzupassen, einschließlich der vorhergesagten Atemanhaltedauer.

In spezielleren Beispielen können Parameter auch die Synchronisation der Bildaufnahme mit dem Atemzyklus der Untersuchungsperson oder anderen physiologischen Prozessen umfassen. In solchen Fällen können die Parameter so konfiguriert werden, dass die Bildgebung während eines Atemanhaltens nach Exspiration oder Inspiration, oder während der optimalen Phase des Atemzyklus oder der physiologischen Aktivität erfolgt, um die bestmögliche Bildqualität zu erzielen.

Die Atemanhaltedauer kann einer Zeitdauer entsprechen, welche die Untersuchungsperson voraussichtlich entsprechend einem Atemanhalteanweisung den Atem anhalten kann.

Das Verfahren kann weiterhin ein Durchführen, unter Verwenden der bestimmten Atemanhaltedauer, zumindest eines Teils des medizinischen Bildgebungsverfahrens an der Untersuchungsperson umfassen, wobei die Untersuchungsperson während des medizinischen Bildgebungsverfahrens angewiesen wird, ihre Atmung zumindest zeitweise anzuhalten.

Die physiologischen Daten können die Untersuchungsperson charakterisieren, während er keine Anweisung zum Anhalten ihrer Atmung erhält.

Die physiologischen Daten können die Untersuchungsperson (zumindest an einem Zeitpunkt / während einer Zeitdauer) vor Beginn des medizinischen Bildgebungsverfahrens charakterisieren.

Die physiologischen Daten könne einen Zustand der Untersuchungsperson während einer Zeitdauer vor Beginn des medizinischen Bildgebungsverfahrens charakterisieren oder repräsentieren, und/oder auf Daten beruhen, welche in der Zeitdauer vorher erhalten wurden.

Das Verfahren kann ein kontinuierliches Erfassen von physiologischen Signalen der Untersuchungsperson während der Positionierung der Untersuchungsperson, Justage-Phase oder vorausgehenden Scans ohne Atemanhalteanweisungen umfassen. Die physiologischen Signale können mindestens eins von einem Atemsignal, EKG-Signal und ein Pulsoxymeter-Signal umfassen.

Das Verfahren kann weiter ein Verarbeiten der erfassten physiologischen Signale mittels eines Analyseverfahrens umfassen, um eine Vorhersage der Atemanhalte-Dauer der Untersuchungsperson nach Inspiration und/oder Exspiration zu generieren.

Das Verfahren kann weiter ein Anpassen von mindestens einem Parameter des Messprotokolls des medizinischen Bildgebungsverfahrens umfassen, beispielsweise um ein MR-Untersuchung basierend auf der vorhergesagten Atemanhalte-Dauer anzupassen, insbesondere um die Anzahl der erforderlichen Teilabschnitte und die Dauer der MR-Untersuchung entsprechend anzupassen.

Physiologische Daten einer Untersuchungsperson können Informationen sein, die den körperlichen Zustand, die Funktionen und Prozesse der Untersuchungsperson beschreiben. Sie können aus Messungen und Beobachtungen von Körpersignalen, Organfunktionen und biologischen Prozessen stammen. Sie können in früheren Messdaten oder Daten aus einer Patientenakte oder von Wearables stammen. Physiologische Daten können den Gesundheitszustand einer Untersuchungsperson beschreiben.

Physiologische Daten können ein beliebiges oder eine beliebige Kombination der Folgenden umfassen: Herzfrequenz, Atemfrequenz oder Atemkurve, Lungenvolumen, Blutdruck, Körpertemperatur, Sauerstoffsättigung im Blut, Messdaten und/oder daraus generierte Datenpunkte und/oder Kennzahlen aus einem oder mehreren der folgenden Messungen:
Elektrokardiogramm (EKG), Elektroenzephalogramm (EEG) und Elektromyogramm (EMG).

Zusätzliche Beispiele für physiologische Daten können die galvanische Hautreaktion (GSR) sein, die Änderungen in der elektrischen Leitfähigkeit der Haut misst, die in der Regel mit Stress oder emotionaler Erregung in Verbindung gebracht werden. Die Pupillengröße, die mit einem Pupillometer gemessen werden kann und auf Lichtverhältnisse, Aufmerksamkeit oder emotionalen Zustand reagiert, kann ebenfalls als physiologische Daten betrachtet werden.

Die Messung von Blutzuckerwerten, die in Milligramm pro Deziliter (mg/dL) oder Millimol pro Liter (mmol/L) ausgedrückt werden können, gehört ebenso zu den möglichen physiologischen Daten wie die Messung von Hormonspiegeln im Blut, beispielsweise Cortisol, Insulin oder Adrenalin.

Schließlich können physiologische Daten auch Informationen über die Aktivität und Bewegung einer Untersuchungsperson enthalten, die mit Beschleunigungsmessern, Gyroskopen oder anderen Bewegungssensoren erfasst werden können. Diese Daten können verwendet werden, um die Mobilität, die körperliche Aktivität oder die Rehabilitation einer Untersuchungsperson zu überwachen und zu bewerten.

Physiologische Daten können damit mit verschiedenen Methoden und Geräten erfasst und oder generiert werden, wie beispielsweise Elektroden, Sensoren, medizinischen Untersuchungstechniken. Diese Daten können in Echtzeit kontinuierlich erfasst und analysiert werden, oder als gespeicherte Daten vorliegen, beispielsweise aus einer Patientendatenbank, abgerufen werden.

Ein Pilot-Tone-Verfahren im Kontext der Magnetresonanztomographie (MRT) kann allgemein als ein Verfahren beschrieben werden, bei dem ein schwaches, kontinuierliches oder periodisches Signal (Pilot-Tone) während der MRT-Untersuchung erzeugt wird. Dieses Signal wird anschließend in der MRT-Datenerfassung detektiert und verwendet, um physiologische Informationen, wie beispielsweise Atembewegungen, Herzschlag oder andere körpereigene Bewegungen der Untersuchungsperson, zu überwachen und zu erfassen. Das Pilot-Tone-Verfahren ermöglicht es, diese physiologischen Informationen während der MRT-Untersuchung zu sammeln, ohne die Bildqualität zu beeinträchtigen oder zusätzliche Hardware- oder Softwarekomponenten hinzufügen zu müssen.

Im Allgemeinen können kontinuierliche Messverfahren physiologischer Daten bei einer Vielzahl von medizinischen Untersuchungsverfahren eingesetzt werden. Im Allgemeinen werden physiologische Informationen überwacht und zu erfasst, ohne die Hauptmessungen oder Untersuchungen zu beeinträchtigen, beispielsweise durch die die Verwendung von Referenzsignalen, Markern oder Synchronisationssignalen, die in Kombination mit Hauptmessungen in bildgebenden Verfahren wie Computertomographie (CT), Ultraschall oder Positronenemissionstomographie (PET) eingesetzt werden.

Während des medizinischen Bildgebungsverfahrens werden Bilddaten einer Untersuchungsperson generiert, insbesondere wird die Untersuchungsperson während des Bildgebungsverfahrens angewiesen, ihre Atmung zumindest zeitweise anzuhalten.

Die offenbarten Techniken sind geeignet zur Verwendung in einem medizinischen Untersuchungsverfahren, z.B. eines (teil) automatisierten Untersuchungsverfahrens, insbesondere in einem medizinischen Bildgebungsverfahren, weiter insbesondere in einem Magnetresonanztomographie (MRT-) oder anderer Bildgebungsverfahren, d.h. unter Verwendung eines entsprechenden medizinischen Untersuchungssystems, insbesondere medizinischen Bildgebungssystems. Entsprechend kann zumindest ein Teil eines medizinischen Untersuchungsverfahrens basierend auf der bestimmten Atemanhaltedauer durchgeführt werden, wobei die Messzeit verkürzt und die Bildqualität erhöht werden kann.

Das Erfassen und Bereitstellen physiologischer Daten kann ohne Atemhalteanweisung erfolgen, in anderen Worten ohne Instruktionen bezüglich Atmung an die Untersuchungsperson. Es ist jedoch auch denkbar, dass der Untersuchungsperson Instruktionen bezüglich ihrer Atmung bereitgestellt werden, die jedoch möglicherweise kein Atemanhalten umfassen, oder ein Atemanhalten ohne vorbestimmte zeitliche Vorgabe umfassen. Beispielsweise können die physiologischen Daten während kontinuierlicher Atmung, vor und/oder während der medizinischen Untersuchung erfasst und/oder generiert werden. Somit kann das Messprotokoll vor Beginn der medizinischen Bildgebung bestimmt werden, und auch während der medizinischen Bildgebung angepasst werden.

Insgesamt erfordert das Verfahren damit keine Verwendung eines dedizierten Vorscans zur Vorhersage der Atemanhaltedauer.

Die Messdaten und/oder Messprotokolle und/oder Parameter und/oder physiologischen Daten können zumindest teilweise in einer Cloud oder im Allgemeinen einer Netzwerkapplikation gespeichert sein. Die Verfahren und Filter können in einer Cloud oder einer verteilten Datenbank implementiert werden.

Unter einer Netzwerkapplikation kann beispielsweise eine dezentral verteilte Datenbank, ein verteiltes Datenbanksystem, eine verteilte Datenbank, eine Peer-to-Peer Applikation, ein verteiltes Speicherverwaltungssystem, eine Blockkette (engl. Blockchain), ein Distributed Ledger, ein verteiltes Speichersystem, ein Distributed Ledger Technology (DLT) basiertes System (DLTS) , ein revisionssicheres Datenbanksystem, eine Cloud, ein Cloud-Service, eine Blockkette in einer Cloud oder eine Peer-to-Peer Datenbank verstanden werden. Beispielsweise kann eine Netzwerkapplikation (oder auch als Netzwerkapplikation bezeichnet) ein verteiltes Datenbanksystem sein, das z.B. mittels einer Blockkette oder einem Distributed Ledger realisiert ist. Auch können beispielsweise unterschiedliche Implementierungen einer Blockkette oder eines DLTS verwendet werden, wie z.B. eine Blockkette oder ein DLTS, die mittels eines Directed Acylic Graph (DAG), eines kryptographischen Puzzles, einem Hashgraph oder einer Kombination aus den genannten Implementierungsvarianten umgesetzt ist. Unter einem verteilten Datenbanksystem oder einer Netzwerkapplikation kann beispielsweise auch ein verteiltes Datenbanksystem oder eine Netzwerkapplikation verstanden werden, von dem/der zumindest ein Teil seiner Knoten und/oder Vorrichtungen und/oder Infrastruktur durch eine Cloud realisiert sind. Beispielsweise sind die entsprechenden Komponenten als Knoten/Vorrichtungen in der Cloud (z.B. als virtueller Knoten in einer virtuellen Maschine) realisiert.

Die Verfahren können vorzugsweise rechnergestützt, d.h. computerimplementiert, realisiert sein.

In einigen Fällen können physiologische Daten auch dazu verwendet werden, um weitere für eine bildgebende Untersuchung kritische Verhaltensweisen von Untersuchungspersonen vorherzusagen, wie beispielsweise die Fähigkeit, unwillkürliche Bewegungen zu kontrollieren, die Stillhaltefähigkeit für bestimmte Zeitintervalle, die Anfälligkeit für Angstzustände oder Klaustrophobie, oder die optimale Herzfrequenz oder den optimalen Blutdruck während der Untersuchung. Beispielsweise kann die Vorhersage von unwillkürlichen Bewegungen, wie Zittern oder Muskelzuckungen, wichtig sein, um die Bildqualität zu verbessern und Artefakte zu reduzieren. Ebenso kann die Fähigkeit der Untersuchungsperson, für einen bestimmten Zeitraum still zu halten, aus physiologischen Daten abgeleitet werden, um geeignete Pausenintervalle während der Untersuchung einzuplanen. Darüber hinaus könnte man auch die Anfälligkeit der Untersuchungsperson für Angst oder Klaustrophobie vorhersagen, um entsprechende Anpassungen bei der Untersuchung vorzunehmen und die Untersuchungspersonkomfort zu erhöhen. Schließlich könnten physiologische Daten auch dazu beitragen, die optimale Herzfrequenz oder den optimalen Blutdruck der Untersuchungsperson während der Untersuchung zu bestimmen, um die Bildgebung in bestimmten Phasen des Herzzyklus zu optimieren und die Gefahr von Bewegungsartefakten zu minimieren.

Zusammenfassend werden Techniken bereitgestellt, um eine Vorhersage der Atemanhalte-Dauern einer Untersuchungsperson während einer MR-Untersuchung bereitzustellen, ohne die Notwendigkeit eines dedizierten Vorscans. Die Vorhersage basiert auf physiologischen Daten, insbesondere kontinuierlich aufgezeichneten physiologischen Signalen wie beispielsweise Atemsignal, EKG-Signalen und Pulsoxymeter-Signalen, die während der Positionierung der Untersuchungsperson, Justage-Phase oder vorausgehenden Scans mit oder ohne Atemanweisungen erfasst werden. Dadurch kann die Untersuchungszeit verkürzt, und durch genauere Vorhersage der Atemanhaltedauern nach Inspiration und Exspiration die Bildqualität erhöht werden.

Obwohl die Erfindung in Bezug auf bestimmte bevorzugte Ausführungsbeispiele gezeigt und beschrieben wurde, werden durch Fachleute nach dem Lesen und Verstehen der Beschreibung Äquivalente und Änderungen vorgenommen werden. Die vorliegende Erfindung umfasst alle derartigen Äquivalente und Änderungen und ist nur durch den Umfang der beiliegenden Ansprüche begrenzt.

Unabhängig vom grammatikalischen Geschlecht eines bestimmten Begriffes sind Personen mit männlicher, weiblicher oder anderer Geschlechteridentität mit umfasst.

## Patentansprüche

1. Verfahren zum Konfigurieren eines Messprotokolls eines medizinischen Bildgebungsverfahrens, umfassend:
- im Vorfeld einer Datenerfassungsphase des medizinischen Bildgebungsverfahrens, Bereitstellen von physiologischen Daten (1) einer Untersuchungsperson (13);
- Vorhersagen, unter Verwenden der physiologischen Daten (1), einer Atemanhaltedauer (6,7) während einer Datenerfassungsphase des medizinischen Bildgebungsverfahrens für die Untersuchungsperson (13), welche einer Zeitdauer entspricht, während der die Untersuchungsperson (13) ihre Atmung auf Anweisung anhalten soll;
- Konfigurieren, unter Verwenden der vorhergesagten Atemanhaltedauer (6,7), zumindest eines Parameters des Messprotokolls.

2. Verfahren nach Anspruch 1, wobei das Bereitstellen der physiologischen Daten (1) umfasst:
- kontinuierliches Erfassen von physiologischen Signalen (2) der Untersuchungsperson (13).

3. Verfahren nach Anspruch 2, wobei die physiologischen Signale (2) in einer Präparationsphase des medizinischen Bildgebungsverfahrens erfasst werden.

4. Verfahren nach Anspruch 2, wobei weitere physiologische Signale während der Datenerfassungsphase des medizinischen Bildgebungsverfahrens erfasst werden, und wobei der zumindest eine Parameter des Messprotokolls basierend auf den weiteren physiologischen Signalen angepasst wird.

5. Verfahren nach einem der Ansprüche 2 bis 4, wobei der Untersuchungsperson (13) für das kontinuierliche Erfassen keine Anweisung zum Anhalten der Atmung bereitgestellt wird.

6. Verfahren nach einem der Ansprüche 2 bis 5, wobei die physiologischen Signale (2) unter Verwenden eines oder mehrerer Sensoren (22,23), die an oder in der Nähe der Untersuchungsperson (13) angeordnet sind, erfasst werden.

7. Verfahren nach einem der Ansprüche 2 bis 5, wobei das medizinische Bildgebungsverfahren ein Magneteresonanztomographie (MRT) -Verfahren ist, und wobei die physiologischen Signale (2) unter Verwendung eines Pilot-Tone-Messverfahrens in einem Frequenzbereich erfasst werden, der außerhalb des für die Erfassung von Bilddaten verwendeten Frequenzbereichs liegt.

8. Verfahren nach einem der vorhergehenden Ansprüche, wobei die physiologischen Daten (1) eins oder mehrere umfassen von: Daten aus einer Patientenakte, Messwerte oder Rohdaten von vorhergehenden Untersuchungen an der Untersuchungsperson (13), und Daten von einem von der Untersuchungsperson (13) am Körper getragenen elektronischen Gerät.

9. Verfahren nach einem der vorhergehenden Ansprüche, wobei das Vorhersagen der Atemanhaltedauer (6,7) für die Untersuchungsperson (13) das Anwenden eines neuronalen Netzwerks (4,5) umfasst, das auf der Grundlage von physiologischen Trainingsdaten der Untersuchungsperson (13) trainiert wurde, wobei eine Eingabe in das neuronale Netzwerk (4,5) zumindest die physiologischen Daten (1) umfasst, und eine Ausgabe des neuronalen Netzwerks (4,5) die Atemanhaltedauer (6,7) umfasst.

10. Verfahren nach Anspruch 9, wobei die physiologischen Daten (1) in Bilddaten (3) konvertiert werden und als Bilddaten (3) in das neuronale Netzwerk (4,5) eingegeben werden.

11. Verfahren nach Anspruch 9 oder 10, wobei das neuronale Netzwerk (4,5) zumindest eines von einem Convolutional Neural Network (CNN) und einem Recurrent Neural Network (RNN) umfasst.

12. Verfahren nach einem der vorhergehenden Ansprüche, wobei die Atemanhaltedauer (6,7) eins umfasst von einer Atemanhaltedauer nach Exspiration (7) und einer Atemanhaltedauer nach Inspiration (6).

13. Verfahren nach einem der vorhergehenden Ansprüche, wobei die physiologischen Signale (2) eines oder mehrere umfassen von einem Atemsignal, einem EKG-Signal und einem Pulsoxymeter-Signal der Untersuchungsperson (13).

14. Verfahren nach einem der vorhergehenden Ansprüche, wobei die physiologischen Daten (1) während eines Positionierens der Untersuchungsperson (13) in einem medizinischen Bildgebungssystem (10), oder während einer Justage des medizinischen Bildgebungssystems (10) für das Bildgebungsverfahren erfasst werden.

15. Medizinisches Bildgebungssystem (10), das ausgebildet ist, um die folgenden Schritte durchzuführen:
- im Vorfeld einer Datenerfassungsphase des medizinischen Bildgebungsverfahrens, Bereitstellen von physiologischen Daten (1) einer Untersuchungsperson (13);
- Vorhersagen, unter Verwenden der physiologischen Daten (1), einer Atemanhaltedauer (6,7) während einer Datenerfassungsphase eines medizinischen Bildgebungsverfahrens für die Untersuchungsperson (13), welche einer Zeitdauer entspricht, während der die Untersuchungsperson ihre Atmung auf Anweisung anhalten soll;
- Konfigurieren, unter Verwenden der vorhergesagten Atemanhaltedauer (6,7), zumindest eines Parameters eines Messprotokolls zur Durchführung einer medizinischen Bildgebung.
